# EUROPEAN PATENT APPLICATION

(11) **EP 3 677 127 A1**
(43) Date of publication of application: **08.07.2020**
(21) Application number: 18850369.2
(22) Date of filing: 30.08.2018
(51) Int. Cl.: A23L 33/18, A23K 10/28, A23K 20/142, A23K 20/163, A23L 33/21, A23L 33/26, A61K 35/20, A61K 38/14, A61K 38/16, A61P 1/14

(54) **INTESTINAL ENVIRONMENT IMPROVEMENT COMPOSITION AND METHOD FOR MANUFACTURING SAME**

(30) Priority: 31.08.2017 JP 2017166833
(71) Applicant: Megmilk Snow Brand Co. Ltd., Sapporo-shi, Hokkaido 065-0043 (JP)
(72) Inventor: FUKUDOME Hirofumi, Sapporo-shi Hokkaido 065-0043 (JP); OGAWA Akihiro, Sapporo-shi Hokkaido 065-0043 (JP); YAMAGUCHI Toshiyuki, Sapporo-shi Hokkaido 065-0043 (JP); ADACHI Noriko, Sapporo-shi Hokkaido 065-0043 (JP); TAGUCHI Yuichi, Sapporo-shi Hokkaido 065-0043 (JP); LI Juan, Sapporo-shi Hokkaido 065-0043 (JP); ITOH Kotaro, Sapporo-shi Hokkaido 065-0043 (JP); KABUKI Toshihide, Sapporo-shi Hokkaido 065-0043 (JP); SAKAI Fumihiko, Sapporo-shi Hokkaido 065-0043 (JP)
(74) Representative: Forstmeyer, Dietmar
(86) International application number: PCT/JP2018/032088
(87) International publication number: WO 2019/044960

(57) **Abstract**

An intestinal environment improvement composition containing a glycopeptide including a peptide having a threonine residue and/or a serine residue and a sugar chain bound to the peptide, in which the glycopeptide has a molecular weight of 500 to 3,000, the sugar chain is bound to a hydroxyl group of the threonine residue or the serine residue of the peptide, a constituent sugar of the sugar chain is at least one selected from the group consisting of sialic acid, N-acetylgalactosamine and galactose. According to the present invention, there are provided a novel intestinal environment improvement composition that has not been present, food products, pharmaceutical products and feed containing the composition.

## Description

### Technical Field

The present invention relates to an intestinal environment improvement composition containing a glycopeptide composition, and intestinal environment improvement foods and drinks, pharmaceutical products and feed containing the composition. The present invention also relates to a glycopeptide composition and a method for producing the glycopeptide composition.

### Background Art

Nutrient in food is digested/absorbed by the digestive tract and circulated in the whole body, and plays an important role in animal growth, development and maintenance of life. In contrast, components (indigestible components) not digested/absorbed by the digestive tract serve various physiological functions through the digestive tract and intestinal bacteria, and thus, are apparently indispensable for maintaining biological homeostasis. Examples of the indigestible components ordinarily known include starchy substances such as resistant starch, indigestible dextrin, and non-starchy substances such as a dietary fiber (e.g., polysaccharide and lignin), an oligosaccharide, a sugar alcohol and a resistant protein. These indigestible components, since industrial production processes thereof have been established, are widely distributed in the market and recognized as food materials contributing to health maintenance/promotion.

In the meantime, use of a sugar chain of a glycoprotein as a new indigestible component, has been expected in recent years (Non Patent Literature 1). The glycoprotein sugar-chain is a sugar chain bound to a protein as it literally means and a component having a structure chemically different from that of known indigestible components, such as a polysaccharide, an oligosaccharide and a sugar alcohol. In particular, mucin, which is a major glycoprotein produced in the digestive tract, has been revealed to be selectively utilized by a representative good bacterium in the intestine, i.e., *Bifidobacterium bifidum* and *Akkermansia muciniphila,* which are suggested to be involved in suppression of obesity and diabetes (Non Patent Literature 2). Mucin produced in the digestive tract is an indigestible substance. Due to this, mucin protects the epithelium of the digestive tract, and has been elucidated to have a regulatory function for intestinal flora as an endogenous indigestible component.

Further, a sugar chain of an N-linked glycoprotein, which is synthesized by a eukaryote such as a yeast, a plant and an animal, has been reported to be selectively utilized by *Bifidobacterium infantis,* and *Bacteroides thetaiotaomicron,* which is a dominant bacterial species in the adult colon (Non Patent Literature 1).

As described above, it has been expected that the glycoprotein sugar-chain is used as a new functional indigestible component. However, since a method for preparing a food material rich in glycoprotein sugar-chain as an indigestible component, industrially in a large scale, has not existed up to the present, the glycoprotein sugar-chain are not distributed in the market.

In the meantime, κ-casein glycomacropeptide (hereinafter sometimes referred to simply as GMP) is a glycopeptide, which is produced by the action of rennet or pepsin on κ-casein of cow's milk, is contained in cheese whey and rennet casein whey. GMP includes a peptide chain of 64 amino acids at the C terminal side of κ-casein and Neu5Acα2-3Galβ1-3(Neu5Acα2-6)GalNAc, Galβ1-3(Neu5Acα2-6)GalNAc, Neu5Acα2-3Galβ1-3GalNAc, Galβ1-3GalNAc or GalNAc bound to the peptide chain (Non Patent Literature 4).

It is expected that GMP can functionally contribute to, e.g., an effect to promote proliferation of bifidobacteria (Non Patent Literature 3) and an effect to improve the intestinal environment (Non Patent Literature 5). Non Patent Literature 3 discloses that GMP is assimilated by bifidobacteria *in vitro.*

Non Patent Literature 4 discloses that feeding GMP to a mouse improves the intestinal environment; for example, a bacterium belonging to the genus *Desulfovibrio*, which proliferates in ulcerative colitis, decreases in number in the intestine, and the levels of intestinal bacterial metabolites beneficial for a host, such as organic acids including acetic acid, propionic acid and butyric acid, increase. It is further reported that anti-inflammatory actions, such as decrease in blood level of inflammatory cytokines and decrease in ratio of inflammatory immune cells in the spleen, are exerted by the intestinal environment improving effect of GMP. Since the quantity of organic acids in the colon and the appendix increases, it is expected that GMP may have, e.g., an effect to promote absorption of a mineral such as calcium in the colon and the appendix (Patent Literatures 1 and 2) and an effect to suppress proliferation of a pathogen (Patent Literature 3).

With respect to these beneficial effects of GMP, it has been considered that particularly the sugar-chain part containing sialic acid of GMP plays an important role in improving the intestinal environment, as prebiotics.

However, GMP contains a peptide chain consisting of 64 amino acids and the ratio of the sugar-chain portion is relatively low, more specifically, the content of sugar is only about 10 wt%. More specifically, in order to take 1 to 10 g of indigestible components (an effective amount of oligosaccharides and dietary fiber) from GMP, 10 to 100 g of GMP must be taken. This is unrealistic as the amount of intake per day. Also, if a food product is produced by adding such a large amount of GMP, GMP has a large effect on flavor and physical properties of the food product and significantly restricts formulation/design thereof.

### Citation List

### Patent Literature

Patent Literature 1: Japanese Patent No. 3575724
Patent Literature 2: Japanese Patent No. 3372499
Patent Literature 3: Japanese Patent No. 2631470

### Non Patent Literature

Non Patent Literature 1: Fujita K. The Metabolic Pathways for the Sugar Chains of Glycoproteins in Bifidobacteria: Prebiotic Glycoproteins Are Growth Substrates for Bifidobacteria. Kagakutoseibutsu, 55(4):242, 2017
Non Patent Literature 2: Ashida H. Interaction between Gut Microbes and Host through Intestical Mucin. Kagakutoseibutsu, 54(12):901-908, 2016
Non Patent Literature 3: Idota T, Kawakami H, Nakajima I. Growth-promoting effects of N-acetylneuraminic acid-containing substances on bifidobacteria. Biosci Biotechnol Biochem. 58:1720-1722, 1994.
Non Patent Literature 4: Saito T., Itoh T. Variations and distributions of O-Glycosidically linked sugar chains in bovine k-casein. J Dairy Sci. 75:1768-1774, 1992.
Non Patent Literature 5: Sawin E. A., De Wolfe T. J., Aktas B., Stroup B. M., Murali S. G., Steele J. L., Ney D.M. Glycomacropeptide is a prebiotic that reduces Desulfovibrio bacteria increases cecal short-chain fatty acids, and is anti-inflammatory in mice. Am J Physiol Gastrointest Liver Physiol. 309:G590-G601, 2015.
Non Patent Literature 6: Robitaille G., Lapointe C., Leclerc D., Britten M. Effect of pepsin-treated bovine and goat caseinomacropeptide on Escherichia coli and Lactobacillus rhmnosus in acidic conditions. J Dairy Sci. 95:1-8, 2012.

### Summary of Invention

### Technical Problem

In the circumstances, it has been desired to develop a glycopeptide component having a sugar chain portion in a concentration at least twice as large as GMP. If such a glycopeptide component can be prepared, it will be a highly effective material per unit amount than GMP (itself), with the result that the addition amount of the component to, e.g., a food product, can be reduced compared to GMP and the effect of the component on flavor and physical properties of a food product can be minimized. The technique for preparing such a substance must be simple and applicable to high-yield food production. However, such a processing technique has not yet been developed (found).

Patent Literature 1 discloses GMP and a GMP-derived peptide as a protective agent against infection; however, whether the effect of the peptide prepared in the literature is higher than that of GMP (itself) and what type of chemical composition was specifically prepared, are not disclosed at all. Patent Literature 1 discloses that GMP is treated with an endopeptidase and the obtained product is subjected to at least one of fractionation means such as gel filtration, ion exchange chromatography and affinity chromatography to separate/obtain the peptide. However, a fractionation method using chromatography has a problem in that the operation is complicated and large amounts of solvent and salt are required for washing and elution. At present, a fractionation method using an ultrafiltration membrane is used; however, a technique for fractionating a peptide having no sugar chain (decomposed with an enzyme) and a peptide having a sugar chain, is not disclosed. The peptide chain of GMP is gradually decomposed into amino acids, peptides and a sugar, each having a molecular weight of 500 or less. Because of this, these substances cannot be fractionated by an ultrafiltration membrane. Non Patent Literature 6 discloses the antibacterial effect/probiotic proliferation effect of a degradant of GMP with pepsin; however, the component involved in the effects is described as 18 residues at the N terminal of the peptide not containing a sugar chain and has a chemically different structure from that of the glycopeptide disclosed by this patent.

An object of the present invention is to provide a glycopeptide composition having a more excellent intestinal environment improving effect than GMP and a method for producing the glycopeptide composition.

### Solution to Problem

The present inventors conducted intensive studies on a method for concentrating a glycopeptide composition from GMP; more specifically, decomposition conditions and fractionation conditions of GMP. As a result, they found a method for preparing a predetermined glycopeptide composition having a constitution shown below. Based on to the finding, the present invention was accomplished.

More specifically, according to an aspect of the present invention, there is provided a method for producing a predetermined glycopeptide composition by digesting GMP with an endoprotease or an exoprotease and subjecting the resultant product to an ultrafiltration membrane (molecular weight cut off: 500 to 3,000).

According to another aspect of the present invention, there is provided a predetermined glycopeptide composition prepared by the aforementioned method and an intestinal environment improvement composition containing the glycopeptide composition as an active ingredient.

More specifically, the present invention has the following constitutions.
<1> An intestinal environment improvement composition containing a glycopeptide including a peptide having a threonine residue and/or a serine residue and a sugar chain bound to the peptide, wherein
   the glycopeptide has a molecular weight of 500 to 3,000,
   the sugar chain is bound to a hydroxyl group of the threonine residue or the serine residue of the peptide, and
   a constituent sugar of the sugar chain is at least one selected from the group consisting of sialic acid, N-acetylgalactosamine and galactose.
<2> The intestinal environment improvement composition according to <1>, wherein the sugar chain of the glycopeptide contains sialic acid and galacto-N-biose.
<3> The intestinal environment improvement composition according to <2>, wherein the molar ratio of the sialic acid and the galacto-N-biose is 1:1 to 2:1.
<4> The intestinal environment improvement composition according to <2>, wherein the total amount of the sialic acid and the galacto-N-biose is 20 wt% or more.
<5> The intestinal environment improvement composition according to any one of <1> to <4>, wherein the number of amino acid residues of a peptide chain of the glycopeptide is 1 to 13.
<6> An intestinal environment improvement composition containing a glycopeptide having a threonine residue and/or a serine residue and a sugar chain bound to a hydroxyl group(s) of the residue(s), wherein
   the sugar chain has at least one structure selected from the group consisting of the following (1) to (5):
   (1) Neu5Acα2-3Galβ1-3(Neu5Acα2-6)GalNAcα1
   (2) Galβ1-3(Neu5Acα2-6)GalNAcα1
   (3) Neu5Acα2-3Galβ1-3GalNAcα1
   (4) Neu5Acα2-3Galβ1-3(O-Ac-Neu5Acα2-6)GalNAcα1
   (5) Neu5Acα2-3Galβ1-3(O-diAc-Neu5Acα2-6)GalNAcα1
<7> The intestinal environment improvement composition according to <6>, wherein the sugar chain has a structure of:
   the above (1); and
   at least one structure selected from the group consisting of the above (2) to (5).
<8> The intestinal environment improvement composition according to any one of <1> to <7>, wherein the composition promotes proliferation of a bifidobacterium.
<9> The intestinal environment improvement composition according to <8>, wherein the bifidobacterium is *Bifidobacterium bifidum.*
<10> The intestinal environment improvement composition according to any one of <1> to <9>, wherein the composition increases the amount of an organic acid in the intestine.
<11> The intestinal environment improvement composition according to <10>, wherein the organic acid is at least one selected from the group consisting of formic acid, acetic acid, propionic acid, butyric acid, lactic acid and succinic acid.
<12> The intestinal environment improvement composition according to any one of <1> to <11>, wherein the glycopeptide is derived from milk.
<13> An intestinal environment improvement food and drink containing the intestinal environment improvement composition according to any one of <1> to <12>.
<14> An intestinal environment improvement pharmaceutical product containing the intestinal environment improvement composition according to any one of <1> to <12>.
<15> An intestinal environment improvement feed containing the intestinal environment improvement composition according to any one of <1> to <12>.
<16> A method for producing a composition containing a glycopeptide including a peptide having a threonine residue and/or a serine residue and a sugar chain bound to the peptide, wherein
   the glycopeptide has a molecular weight of 500 to 3,000,
   the sugar chain is bound to a hydroxyl group of the threonine residue or the serine residue of the peptide, and
   a constituent sugar of the sugar chain is at least one selected from the group consisting of sialic acid, N-acetylgalactosamine and galactose,
   the method including the following steps of:
   (1) treating κ-casein glycomacropeptide with an endoprotease or an exoprotease, and
   (2) subjecting a product obtained by the treatment in the above step to ultrafiltration (molecular weight cut off: 500 to 3,000).
<17> A glycopeptide composition containing a glycopeptide including a peptide having a threonine residue and/or a serine residue and a sugar chain bound to the peptide, wherein
   the glycopeptide has a molecular weight of 500 to 3,000,
   the sugar chain is bound to a hydroxyl group of the threonine residue or the serine residue of the peptide, and
   a constituent sugar of the sugar chain is at least one selected from the group consisting of sialic acid, N-acetylgalactosamine and galactose.
<18> The glycopeptide composition according to <17>, wherein the sugar chain of the glycopeptide contains sialic acid and galacto-N-biose.
<19> The glycopeptide composition according to <17> or <18>, wherein the sugar chain of the glycopeptide consists of sialic acid and galacto-N-biose.
<20> The glycopeptide composition according to any one of <17> to <19>, wherein the molar ratio of the sialic acid and the galacto-N-biose is 1.5:1 to 2:1.
<21> The glycopeptide composition according to any one of <17> to <20>, wherein the total amount of the sialic acid and the galacto-N-biose is 20 wt% or more.
<22> The glycopeptide composition according to any one of <17> to <21>, wherein the number of amino acid residues in the peptide chain of the glycopeptide is 1 to 13.
<23> A glycopeptide composition containing a glycopeptide having a threonine residue and/or a serine residue and a sugar chain bound to a hydroxyl group(s) of the residue(s), wherein the sugar chain has at least one structure selected from the group consisting of the following (a) to (e):
   (a) Neu5Acα2-3Galβ1-3(Neu5Acα2-6)GalNAcα1
   (b) Galβ1-3(Neu5Acα2-6)GalNAcα1
   (c) Neu5Acα2-3Galβ1-3GalNAcα1
   (d) Neu5Acα2-3Galβ1-3(O-Ac-Neu5Acα2-6)GalNAcα1
   (e) Neu5Acα2-3Galβ1-3(O-diAc-Neu5Acα2-6)GalNAcα1
<24> The glycopeptide composition according to <23>, wherein the sugar chain has a structure of the above (a) and at least one structure selected from the group consisting of the above (b) to (e).

### Advantageous Effects of Invention

According to the present invention, it is possible to provide a glycopeptide composition having a more excellent intestinal environment improving effect than GMP and a method for producing the glycopeptide composition by a simple method applicable to food products.

### Brief Description of Drawings

[Figure 1] The figure shows a chart showing the analysis results by size exclusion chromatography (SEC) of glycopeptides.
[Figure 2] The figure is a graph showing the results of assimilation tests of glucose, GMP and a glycopeptide by bifidobacteria.
[Figure 3] The figure is a graph showing the weight of the contents of the mouse appendix in a GMP administration group and a glycopeptide administration group.
[Figure 4] Figure 4A is a graph showing the amounts of organic acids in the contents of the mouse appendix in a GMP administration group and a glycopeptide administration group. Figure 4B is a graph showing the total amount of organic acids in the contents of the mouse appendix in a GMP administration group and a glycopeptide administration group.

### Description of Embodiments

A composition containing a glycopeptide composition of the present invention as an active ingredient and having an intestinal environment improving effect, and foods and drinks, pharmaceutical products and feed containing the glycopeptide composition as an active ingredient and having an intestinal environment improving effect, will be more specifically described below.

### (Glycopeptide composition)

The glycopeptide composition of the present invention can be a glycopeptide including a peptide having a threonine residue and/or a serine residue and a sugar chain bound to the peptide, wherein the glycopeptide has a molecular weight of 500 to 3,000 and the sugar chain is bound to a hydroxyl group of a threonine residue or a serine residue, a constituent sugar of the sugar chain is at least one selected of the group consisting of sialic acid, N-acetylgalactosamine and galactose. The lower limit of the molecular weight is further preferably 1,000 or more.

In the glycopeptide composition of the present invention, a sugar chain preferably consists of sialic acid and galacto-N-biose (Galβ1-3GalNAc), and further preferably, has sialic acid and galacto-N-biose in a molar ratio within the range of 1: 1 to 2: 1 and contains sialic acid and galacto-N-biose in a total amount of 20 wt% or more.

Also, in the glycopeptide composition of the present invention that can be used, the peptide chain consists of about 1 to 13 of amino acid residues. Further, the glycopeptide composition of the present invention that can be used contains acetylated sialic acid.

An embodiment of the glycopeptide composition according to the invention of the present application is a composition containing a sialyl-glycopeptide having a threonine residue and/or a serine residue and a sugar chain bound to a hydroxyl group of the residue(s), in which the sugar chain has at least one structure selected from the group consisting of the following (1) to (5):
(1) Neu5Acα2-3Galβ1-3(Neu5Acα2-6)GalNAcα1
(2) Galβ1-3(Neu5Acα2-6)GalNAcα1
(3) Neu5Acα2-3Galβ1-3GalNAcα1
(4) Neu5Acα2-3Galβ1-3(O-Ac-Neu5Acα2-6)GalNAcα1
(5) Neu5Acα2-3Galβ1-3(O-diAc-Neu5Acα2-6)GalNAcα1

Another embodiment of the glycopeptide composition according to the invention of the present application is a composition containing a sialyl-glycopeptide, in which the sugar chain has the structure represented by the following (1) and at least one selected from the group consisting of structures represented by the following (2) to (5), in combination.
(1) Neu5Acα2-3Galβ1-3(Neu5Acα2-6)GalNAcα1
(2) Galβ1-3(Neu5Acα2-6)GalNAcα1
(3) Neu5Acα2-3Galβ1-3GalNAcα1
(4) Neu5Acα2-3Galβ1-3(O-Ac-Neu5Acα2-6)GalNAcα1
(5) Neu5Acα2-3Galβ1-3(O-diAc-Neu5Acα2-6)GalNAcα1

The glycopeptide composition of the present invention can be obtained by hydrolyzing, e.g., GMP with, e.g., an enzyme.

GMP is a C-terminal peptide containing a sugar chain of κ-casein and correspond to 106-169 residues of κ-casein in cow's milk and has a molecular weight of about 7,000. As GMP serving as a raw material for the glycopeptide composition of the present invention, any GMP can be used as long as it can be obtained from whey of milk of a farm animal of not only a cow but also a goat and a sheep.

Alternatively, the glycopeptide composition of the present invention can be prepared by hydrolyzing an extract from a microorganism, a plant or an animal organ with, e.g., an enzyme. Further, a glycopeptide composition chemically synthesized or prepared by use of a transgenic organism may be used.

### (Method for producing glycopeptide composition)

With respect to the method for producing the glycopeptide composition of the present invention, an embodiment thereof will be described below. The glycopeptide composition of the present invention can be obtained by hydrolyzing, for example, GMP. Thus, first, an embodiment of a method for producing GMP will be described.

A composition containing GMP can be prepared by a method disclosed in Japanese Patent No. 2673828 or Japanese Patent Laid-Open No. H6-25297. More specifically, a method for preparing GMP by, first, controlling the pH of a GMP-containing material derived from a milk such as cheese whey, a whey protein concentrate and deproteinized cheese whey, to be less than 4, subjecting the prepared material to ultrafiltration using a membrane (molecular weight cut off: 10,000 to 50,000) to obtain a permeate (preferably controlling the pH of a permeable membrane again to be 4 or more) and carrying out desalting by use of a membrane (molecular weight cut off: 50,000 or less) followed by concentrating the permeate, is mentioned. Alternatively, a method for preparing a composition having a high GMP content by controlling milk whey so as to have pH 6.0 or more, treating the milk whey with heat at 40 to 79°C, subjecting it to a treatment with a membrane to remove whey protein, recovering GMP in a concentrate, controlling the concentrate obtained so as to have pH 5.5 or less, subjecting it again to a treatment with a membrane and recovering GMP in the permeate, can be mentioned.

The amount of GMP in the composition obtained can be analyzed by urea-SDS electrophoresis disclosed, for example, in Examples of Patent Literature 2.

Then, GMP thus obtained is subjected to a treatment step with an endoprotease or an exoprotease and a treatment step of subjecting the GMP treated in the above step to ultrafiltration (molecular weight cut off: 500 to 3,000). Through the steps, the glycopeptide composition of the present invention can be produced.

The type of protease for producing a glycopeptide is not particularly limited as long as it can hydrolyze a peptide bond to provide a glycopeptide having the molecular weight described in the above section "(Glycopeptide composition)", and a sugar chain. A single type or a plurality of endoproteases and exoproteases can be used. Preferably, for example, an enzyme(s) applicable to production of food products and pharmaceutical products may be used; more specifically, Actinase E (Kaken Pharma Co., Ltd.), Actinase AS (Kaken Pharma Co., Ltd.), Nucleicin (HBI), Orientase AY (HBI), SUMIZYME FP (Shin Nippon Chemical Co., Ltd.), SUMIZYME SPP-G (Shin Nippon Chemical Co., Ltd.), Protease A (Amano Enzyme Inc.) and Peptidase R (Amano Enzyme Inc.) can be used singly or in combination.

### (Evaluation of intestinal environment improving effect of glycopeptide composition)

The intestinal environment improving effect by the glycopeptide composition of the present invention is evaluated based on comparison of the amount of organic acids in the contents of the intestine or feces between the case where a glycopeptide composition is administered and the case where the glycopeptide is not administered. If the amount of organic acids is higher in the administration case than the non-administration case, it can be evaluated that the composition has the intestinal environment improving effect of the present invention. Further preferably, the amount of organic acids in the contents of the intestine or feces is compared to the case of administering GMP. If the amount of organic acids is higher in the case of administering the glycopeptide composition than that in case of administering GMP, it can be evaluated that the intestinal environment improving effect of the present invention is remarkable.

Also, in an *in-vitro* bifidobacterium proliferation test, when the proliferative ability of a bifidobacterium in the medium is compared between the case where a glycopeptide composition is added as a sugar source in a medium and the case where the glycopeptide composition is not added (without sugar), if the proliferative ability of the bifidobacterium in the presence of a glycopeptide composition is higher than that in the absence of a glycopeptide composition, it can be evaluated that the glycopeptide composition has the intestinal environment improving effect of the present invention. Further preferably, the proliferative ability of a bifidobacterium is compared to the case of administering GMP. If the proliferative ability of a bifidobacterium is higher in the case of administering the glycopeptide composition than that in case of administering GMP, it can be evaluated that the intestinal environment improving effect of the present invention is remarkable.

### (Food/drink, pharmaceutical product and feed containing glycopeptide composition)

The glycopeptide composition obtained by the production method of the present invention can be directly used as a material or a raw material for foods and drinks. The foods and drinks may be prepared in accordance with routine methods for food products except that a composition containing a glycopeptide is added.

Accordingly, the glycopeptide composition of the present invention may be blended in an effective amount in any food and drink and may be added in a raw material for a food and drink during a production step. Examples of the foods and drinks include, but are not particularly limited to, dairy products such as cheese, fermented milk, dairy product/lactic acid bacteria beverage, lactobacillus beverage, butter and margarine; drinks such as milk drink, juice drink and soft drink; egg products such as jelly, candy, pudding and mayonnaise; sweets/bread such as butter cake; milk powders; baby foods; and nutrition compositions.

The foods and drinks containing an effective amount of the glycopeptide composition produced as described above are provided as foods and drinks having an intestinal environment improving effect.

The glycopeptide composition obtained by the production method of the present invention can be directly used as a raw material for pharmaceutical products. The pharmaceutical products may be prepared in accordance with the routine methods for tablets, capsules, powders and syrups except that the glycopeptide composition is added.

Accordingly, pharmaceutical products containing the glycopeptide composition of the present invention as an active ingredient can be prepared by appropriately blending pharmaceutically acceptable additives such as an excipient, a stabilizer and a flavoring agent. Other than this, the glycopeptide composition can be directly dried and used as a powdered medicine. Alternatively, the glycopeptide composition can be produced into a preparation by blending an excipient, a binder, a disintegrant, a lubricant, a flavoring agent, a suspending agent, a coating agent and an optional drug as long as they do not interfere with the intestinal environment improving effect. As the dosage form, tablets, capsules, granules, powders and syrups can be employed.

The pharmaceutical products containing an effective amount of the glycopeptide composition and produced as described above are provided as pharmaceutical products having an intestinal environment improving effect.

The glycopeptide composition obtained by the production method of the present invention can be directly used as a raw material for feed. The feed may be prepared in accordance with routine methods for feed except that the glycopeptide composition is added.

Accordingly, as with the foods and drinks, the glycopeptide composition of the present invention can be blended in an effective amount in any feed and added in a raw material for feed during a production step.

The feed containing an effective amount of the glycopeptide composition thus produced is provided as feed having an intestinal environment improving effect.

### (Amount of intake of glycopeptide composition)

If a composition having an intestinal environment improving effect is produced by blending the glycopeptide composition of the present invention with materials for, e.g., a food and drink, a pharmaceutical product and feed or processed products of these materials, the blending ratio of the glycopeptide composition is not particularly limited and may be appropriately controlled in consideration of easiness in production and in accordance with a preferable daily dose.

The daily dose is independently determined in consideration of, e.g., the symptom and age of the administration target. In the case of an adult human, the glycopeptide composition may be taken satisfactorily in a dose of 0.1 g or more per day, preferably 1 g or more and further preferably 10 g or more.

### [Example 1] Method for preparing the glycopeptide composition of the present invention

### 1. Method for producing GMP

A whey protein concentrate (Sun Lacto N-2, manufactured by Taiyo Kagaku Co., Ltd.) (1 kg) was dissolved in water (50 L) of 50°C and pH thereof was controlled with concentrated hydrochloric acid to be 3.5. This solution was subjected to ultrafiltration using an ultrafiltration membrane (molecular weight cut off: 20,000) (GR61PP, manufactured by DDS) at 50°C, a pressure of 0.4 MPa and an average permeate flow rate of 52.4 L/m²·h. Ultrafiltration was continuously carried out while adding water (40 L) of 50°C to the concentrate when the volume of the permeate reached 40 L. Continuous operation was carried out in the manner mentioned above to obtain a permeate of 160 L.

To the resultant permeate, a 25% caustic soda was added to adjust pH to be 7.0. The permeate was subjected again to ultrafiltration using the same ultrafiltration membrane in the same conditions until the volume of a concentrate reached 5 L to desalt and concentrate. Subsequently, water of 50°C was added. While the volume of the concentrate was always maintained at 10 L, diafiltration was carried out by using the same ultrafiltration membrane in the same conditions as above. In this manner, the permeate was further desalted. At the time when the volume of the permeate reached 80 L by the diafiltration, addition of water to the concentrate was terminated. The permeate was concentrated by ultrafiltration until the volume of the concentrate reached 2 L. The concentrate was dried to obtain GMP (54 g).

### 2. Method for producing glycopeptide composition

Using GMP obtained in Section 1, a 5% GMP solution (30 Kg) was prepared. The GMP solution, to which an endoprotease (Actinase AS, manufactured by Kaken Pharma Co., Ltd.) was added, was allowed to react at 50°C for 6 hours. The resultant GMP solution was subjected to ultrafiltration using an ultrafiltration membrane (molecular weight cut off: 1,000). To the concentrate, water was added and the solution mixture was subjected to ultrafiltration. In this manner, continuous ultrafiltration (diafiltration) was carried out. After 5-fold hydro-concentration was carried out, a concentrate fraction was obtained. The concentrate was dried to obtain glycopeptide composition A (271.7 g). 3. Method for producing glycopeptide composition by combination of two types of enzymes

Using GMP obtained in the above Section 1, a 5% GMP solution (30 Kg) was prepared. The GMP solution, to which an endoprotease (Orientase AY, manufactured by HBI) was added, was allowed to react at 50°C for 4 hours. Thereafter, an exoprotease (Peptidase R, manufactured by Amano Enzyme Inc.) was added and a reaction was carried out at 37°C for 2 hours. The resultant solution was subjected to ultrafiltration using an ultrafiltration membrane (molecular weight cut off: 1,000). To the concentrate, water was added and the solution mixture was subjected to ultrafiltration. In this manner, continuous ultrafiltration (diafiltration) was carried out. After 5-fold hydro-concentration was carried out, a concentrate fraction was obtained. The concentrate was dried to obtain glycopeptide composition B (312.6 g).

### [Example 2] Treatment of glycopeptide composition with sialidase

To glycopeptide composition A prepared in Example 1, a 100 mM acetate buffer (pH 5.0) containing 2 mM calcium chloride was added to prepare a 20 mg/mL solution thereof. To the solution, sialidase (derived from *Clostridium perfringens*: Sigma, N2876-25UN) was added so as to be 10 U/mL. The resultant solution was reacted at 37°C for 20 hours. A blank (the enzyme was not added) was reacted in the same conditions (without adding the enzyme). After completion of the reaction, boiling was carried out for 5 minutes to terminate the reaction. The resultant reaction solutions were subjected to size exclusion chromatography (SEC). In the SEC analysis, L-2000 (HITACHI) system equipped with two TSKgel G3000PW (Tohso Corporation) was used and absorption at UV 214 nm was detected. As a mobile phase, a 40% acetonitrile solution containing 0.1% of trifluoro acetic acid was used and isocratic elution was carried out at a flow velocity of 0.3 mL/min for 120 minutes at room temperature.

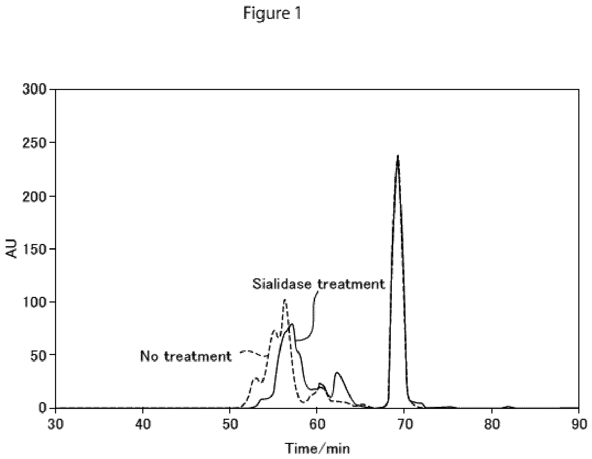

The results of SEC analysis are shown in Figure 1. A big peak was observed near 70 minutes; however, this peak is derived from the acetate buffer used in the enzymatic reaction.

The major peaks of the glycopeptide composition were observed from 50 minutes to 60 minutes. The peaks of the glycopeptide composition treated with sialidase shifted in whole toward the low molecular side. From this, it was found that the main substance in the prepared glycopeptide composition A is a compound containing sialic acid.

### [Example 3] Structure analysis of glycopeptide composition by LC/MS

For LC/MS analysis, an ion-trap system mass spectrometer LTQ Velos (Thermofisher Scientific) connected to high-performance liquid chromatography (HPLC) ParadigmMS2 (Michrom Bioresources) was used (LC-ESI-IT MS) and was equipped with a separation column, i.e., InertSustain C18 (φ0.2 mm × 150 mm, GL Sciences Inc.). For the mobile phase, a 2% acetonitrile solution (A solution) containing 0.1% of formic acid and a 90% acetonitrile solution (B solution) containing 0.1% formic acid were used. Glycopeptide composition A was dissolved in a 50% acetonitrile-0.1% formic acid solution to prepare a 100 µg/mL solution. An aliquot (25 µL) of the solution was taken and subjected in HPLC. The flow rate of the mobile phase was 2 µL/min. In the period from 0 to 90 minutes after introduction of a sample, the ratio of B solution was linearly increased from 0% to 45%. MS measurement (m/z 400-2000, positive mode) and MS/MS measurement were carried out by a mass spectrometer.

In the MS/MS measurement, 5 types of ions in descending order from MS intensity value were automatically selected as precursor ions and destroyed at a collision energy of 35 V, and then, the m/z values of the resultant product ions were detected. The sugar chain having a difference of 292 in m/z value between the precursor ions and product ions was regarded as Neu5Ac; a difference of 334 as N,O-diacetylneuraminic acid (O-Ac-Neu5Ac), a difference of 376 as N,O,O-triacetylneuraminic acid (O,O-diAc-Neu5Ac), a difference of 203 as GalNAc; and a difference of 162 as Gal. Based on the patterns of product ions, the structures of sugars were determined. The m/z value of ions in the case where a sugar chain was completely dissociated was presumably derived from ions in the case where protons were added to a peptide. On the presumption, the peptide sequence corresponding to the m/z value was searched from GMP sequences. In this manner the structure of a glycopeptide was estimated.

With respect to glycopeptide composition A prepared in Example 1, the sugar structure of the glycopeptide contained in the glycopeptide composition was estimated based on the MS spectrum and MS/MS spectrum obtained in LC/MS analysis.

Table 1 shows the sugar structures of the glycopeptides determined in the LC/MS analysis. In the analysis result, it was found that any one of glycopeptides detected includes a peptide and a sialic acid-containing sugar chain bound to the peptide. Most of the glycopeptides detected had a sugar chain structure of Neu5Acα1-3Galβ1-3(Neu5Acα1-6)GalNAc (Glycan type 3, 4, 5, 6, 7) having two sialic acid molecules bound therein; however, Neu5Acα1-3Galβ1-3GalNAc (Glycan type 1) and Galβ1-3(Neu5Acα1-6)GalNAc (Glycan type 2) having a single sialic acid molecule, were detected. Further, O-acetyl forms of sialic acid, i.e., N,O-diacetylneuraminic acid (O-Ac-Neu5Ac, Glycan type 4), and N,O,O-triacetylneuraminic acid (O-diAc-Neu5Ac, Glycan type 5) were detected. Also, glycopeptides (Glycan type 6, 7) formed of a single peptide chain having two sugar chains bound thereto were detected.

The peptide chains detected from MS/MS spectral analysis were all peptide chains contained in cow's glycomacropeptides. In the peptide chains of the all glycopeptides detected contained at least one serine or threonine residue. The shortest peptide-chain length was the length corresponding to a single residue; whereas, the longest peptide-chain length was the length corresponding to 13 residues. The molecular weights of the peptide chains detected fell within the range of 745 to 2928.

### [Table 1]

**(Table 1) Structure analysis of glycopeptide by LC/MS**

| Glycan type on peptide | Glycan chain | Glycan MW |
|---|---|---|
| 1 | Neu5Acα2-3Galβ1-3GalNAcα1-S/T | 657 |
| 2 | Galβ1-3(Neu5Acα2-6)GalNAcα1-S/T | 657 |
| 3 | Neu5Acα2-3Galβ1-3(Neu5Acα2-6)GalNAcα1-S/T | 948 |
| 4 | Neu5Acα2-3Galβ1-3(*O*-Ac-Neu5Acα2-6)GalNAcα1-S/T | 990 |
| 5 | Neu5Acα2-3Galβ1-3(*O*-diAc-Neu5Acα2-6)GalNAcα1-S/T | 1032 |
| 6 | Neu5Acα2-3Galβ1-3(Neu5Acα2-6)GalNAcα1-S/T | 948+657 |
| | Neu5Acα2-3Galβ1-3GalNAcα1-S/T | |
| 7 | Neu5Acα2-3Galβ1-3(Neu5Acα2-6)GalNAcα1-S/T | 948+948 |
| | Neu5Acα2-3Galβ1-3(Neu5Acα2-6)GalNAcα1-S/T | |

### [Example 4] Constituent sugar and content ratio of glycopeptide composition

A glycopeptide composition is constituted of a sugar chain and a peptide chain. It is considered that a constituent sugar of the glycopeptide composition and the content of the sugar are important for improving the intestinal environment.

As shown in Example 3, it was found that the sugar chain of a glycopeptide composition is constituted of sialic acid (N-acetylneuraminic acid) and galacto-N-biose (GNB: Galb1-3GalNAc). Accordingly, as a next step, the amounts of sialic acid and galacto-N-biose in the glycopeptide composition were measured, and the content ratio of them were checked.

The amounts of sialic acid in the GMP and glycopeptide compositions prepared in Example 1 were measured by the method described below. More specifically, to measure the amount of sialic acid, a sample solution (100 µL), water (800 µL) and a 1 N sulfuric acid solution (100 µL) were added in a screw-top test tube and heated by use of a block heater at 80°C for 45 minutes. After cooling, the reaction solution was neutralized with the same amount of 100 mM sodium hydroxide, and then, insoluble matter was removed by a 0.45-µm filter. The content of sugar in the resultant reaction solution was measured by DIONEX ICS-5000DP system equipped with Carbopak PA1 column.

In order to separate galacto-N-biose from the GMP and glycopeptide compositions prepared in Example 1, first, the GMP and glycopeptide compositions were treated with sialidase in accordance with the method described in Example 2. The GMP and glycopeptide compositions treated with sialidase were sequentially treated with O-glucanase (endo-α-N-acetylgalactosaminidase) to separate galacto-N-biose from the glycopeptide compositions. The galacto-N-biose separated was measured by DIONEX ICS-5000DP system equipped with Carbopak PA1 column. The results are shown in Table 2.

The contents of sialic acid (N-acetylneuraminic acid: Neu5Ac) and galacto-N-biose (GNB: Galb1-3GalNAc) were higher in the glycopeptide compositions than in GMP. Also, it was found that the total sugar content of sialic acid and galacto-N-biose in glycopeptide composition A is 3.9 times as high as in GMP; and the total sugar content in glycopeptide composition B is 2.8 times as high as in GMP. It was apparent that the sugar chain of GMP is concentrated.

### [Table 2]

**(Table 2) Sugar composition in GMP and glycopeptide compositions**

| | | Sugar content (wt%) | Molar ratio (GNB=1) |
|---|---|---|---|
| **GMP** | **GNB** | **5.1** | **1.00** |
| | **Neu5Ac** | **6.0** | **1.46** |
| | **Total** | **11.1** | **-** |
| Glycopeptide composition A | **GNB** | **17.4** | **1.00** |
| | **Neu5Ac** | **26.1** | **1.86** |
| | **Total** | **43.5** | **-** |
| Glycopeptide composition B | **GNB** | **13.4** | **1.00** |
| | **Neu5Ac** | **18.0** | **1.67** |
| | **Total** | **31.4** | - |

### [Experiment 1] Study of assimilation of glycopeptide composition by bifidobacterium

Assimilation of GMP or glycopeptide composition by bifidobacterium was checked.

### 1. Experimental method

For the assimilation test, PY culture medium (200 mM PIPES, pH 6.7, 2g/L peptone, 2 g/L BBL Trypticase Peptone, 2g/L Bacto Yeast Extract, 8 mg/L CaCL2, 19.2 mg/L MgSO4 7H2O, 80 mg/L NaCl, 4.9 mg/L hemin, 0.5 g/L L-Cycteine hydrochloride, 100 µg/L vitamin K1, autoclaved 115°C, 15 min) was used. To PY culture medium containing no sugar source, sugar sources, i.e., 0.5% of glucose (Glc), 1.5% of GMP, and 1.5% of glycopeptide composition A prepared in Example 1 were added.

As the bifidobacteria, *Bifidobacterium bifidum* JCM1254, *Bifidobacterium bifidum* JCM7004 and *Bifidobacterium bifidum* SBT10550 were used and the bifidobacteria were cultured in GAM culture medium previously containing 1% glucose for 18 hours. The culture solutions were inoculated in the PY culture mediums in a concentration of 0.1%. The PY culture mediums having the bacteria inoculated, respectively were seeded in a 96-well microplate(s) in a rate of 200 µL/well and cultured in anaerobic conditions at 37°C for 72 hours. The proliferation of the bacterial cells was evaluated based on turbidity (OD 660 nm).

### 2. Test results

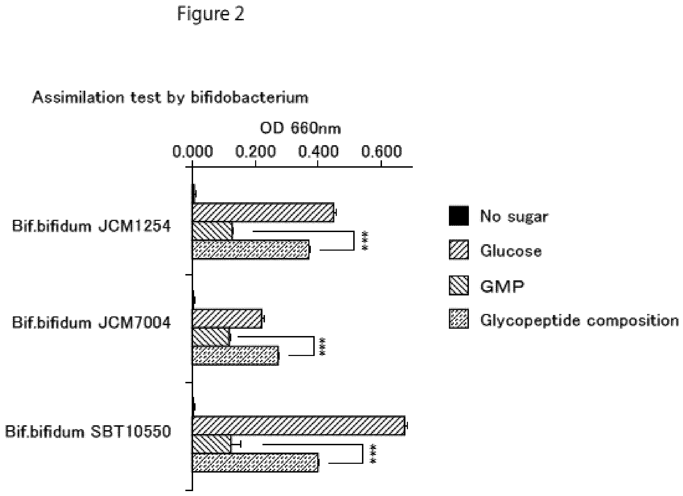

Bifidobacteria did not proliferate in mediums containing no sugar sources; however, bifidobacteria proliferated in the segments containing glucose, GMP and a glycopeptide composition. Also, All 3 bacterial strains highly proliferated statistically significantly in the segment containing the glycopeptide composition than GMP (Figure 2). In Figure 2, the symbol mark "***" represents statistical significance compared to GMP (p < 0.001) .

Accordingly, it was apparent that a glycopeptide composition has an action to promote proliferation of bifidobacteria.

### [Experiment 2] Study on intestinal organic acid increasing effect of glycopeptide composition

### 1. Test method

Mice were divided into three groups. These groups were fed with Normal feed (control group), feed containing 10% of GMP (GMP group) and feed containing 10% of glycopeptide composition A prepared in Example 1 (glycopeptide composition group), respectively, for three weeks. The weights of the contents in appendixes of the mice and the amounts of organic acids in the contents of the appendixes were measured. Since the site mainly responsible for fermenting indigestible components in a human is the colon; however, the site in a mouse is the appendix, the contents in the appendix were measured in this experiment. Thus, experimental results show the weights of the appendix contents and the amount of organic acids; however, these weights and organic-acid amount do not limitedly represent the results of actions in the appendix and are replaceable for the weight of the contents in the human's colon, (which is a site mainly responsible for fermentation in a human) and contents of the large intestine or amount of organic acids in feces.

### 2. Test results

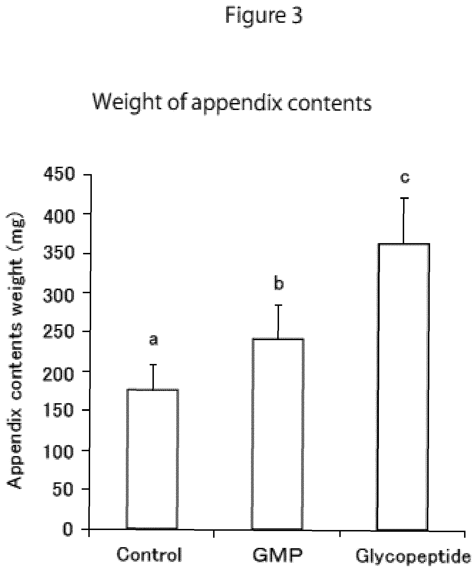

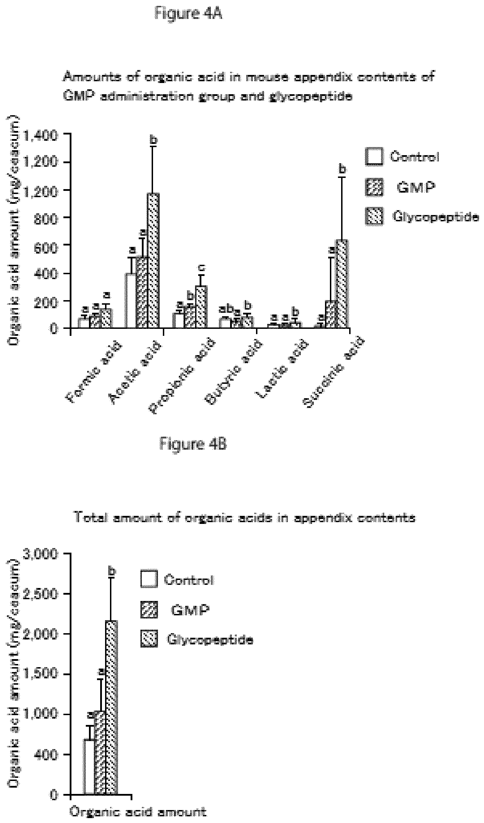

The weight of the appendix contents of a glycopeptide composition group was significantly high compared to those of the control group and the GMP group (Figure 3). The amount of organic acids in the appendix contents was measured. As a result, the amounts of formic acid, acetic acid, propionic acid, butyric acid, lactic acid, succinic acid and the total organic acids in the glycopeptide composition group were significantly high, compared to those of the control group and the GMP group (Figure 4A, Figure 4B).

Thus, it was apparent that the glycopeptide composition has a more excellent organic acid producibility than GMP. Reference symbols a, b and c in Figures 3 and 4 indicate statistical significance between the segments attached with different reference symbols (p < 0.05).

### [Example 5] Production of supplement

To a powder of the glycopeptide composition (30 g) obtained in Example 1, a mixture (40 g) of equal amounts of vitamin C and citric acid, granulated sugar (100 g) and a mixture (60 g) of equal amounts of corn starch and lactose were added and stirred. The mixture was stuffed in stick pouches to prepare a supplement for improving the intestinal environment according to the present invention.

### [Example 6] Production of drink

Raw materials were mixed in accordance with the formulation shown in Table 3, stuffed in containers and sterilized with heat to prepare drinks for improving the intestinal environment according to the present invention.

### [Table 3]

**(Table 3)**

| | Content (wt%) |
|---|---|
| Glycopeptide composition powder of Example 1 | 2.5 |
| Sugar | 7.5 |
| Citric acid | 0.6 |
| Apple juice | 10.0 |
| Water | 79.4 |

### [Example 7] Production of pharmaceutical product (encapsulated formulation)

Raw materials were mixed in accordance with the formulation shown in Table 4 and granulated to prepare a granulated powder, which was stuffed in empty capsules by 10 mg per capsule to prepare an encapsulated formulation containing a pharmaceutical product for improving the intestinal environment according to the present invention.

### [Table 4]

**(Table 4)**

| | Content (wt%) |
|---|---|
| Glycopeptide composition powder of Example 1 | 20.0 |
| Lactose | 24.5 |
| Soluble starch | 55.0 |
| Magnesium stearate | 0.5 |

### Industrial Applicability

According to the present invention, it became possible to provide a novel intestinal environment improvement composition containing a glycopeptide composition as an active ingredient, and intestinal environment improvement foods and drinks, pharmaceutical products and feed containing a glycopeptide composition as an active ingredient.

## Claims

1. An intestinal environment improvement composition containing a glycopeptide comprising a peptide having a threonine residue and/or a serine residue and a sugar chain bound to the peptide, wherein
the glycopeptide has a molecular weight of 500 to 3,000,
the sugar chain is bound to a hydroxyl group of the threonine residue or the serine residue of the peptide, and
a constituent sugar of the sugar chain is at least one selected from the group consisting of sialic acid, N-acetylgalactosamine and galactose.

2. The intestinal environment improvement composition according to Claim 1, wherein the sugar chain of the glycopeptide contains sialic acid and galacto-N-biose.

3. The intestinal environment improvement composition according to Claim 2, wherein the molar ratio of the sialic acid and the galacto-N-biose is 1:1 to 2:1.

4. The intestinal environment improvement composition according to Claim 2, wherein the total amount of the sialic acid and the galacto-N-biose is 20 wt% or more.

5. The intestinal environment improvement composition according to any one of Claims 1 to 4, wherein the number of amino acid residues of a peptide chain of the glycopeptide is 1 to 13.

6. An intestinal environment improvement composition containing a glycopeptide having a threonine residue and/or a serine residue and a sugar chain bound to a hydroxyl group of the residue(s), wherein
the sugar chain has at least one structure selected from the group consisting of the following (1) to (5):
(1) Neu5Acα2-3Galβ1-3(Neu5Acα2-6)GalNAcα1
(2) Galβ1-3(Neu5Acα2-6)GalNAcα1
(3) Neu5Acα2-3Galβ1-3GalNAcα1
(4) Neu5Acα2-3Galβ1-3(O-Ac-Neu5Acα2-6)GalNAcα1
(5) Neu5Acα2-3Galβ1-3(O-diAc-Neu5Acα2-6)GalNAcα1

7. The intestinal environment improvement composition according to Claim 6, wherein the sugar chain has a structure of:
the above (1); and
at least one structure selected from the group consisting of the above (2) to (5).

8. The intestinal environment improvement composition according to any one of Claims 1 to 7, wherein the composition promotes proliferation of a bifidobacterium.

9. The intestinal environment improvement composition according to Claim 8, wherein the bifidobacterium is *Bifidobacterium bifidum.*

10. The intestinal environment improvement composition according to any one of Claims 1 to 9, wherein the composition increases the amount of an organic acid in the intestine.

11. The intestinal environment improvement composition according to Claim 10, wherein the organic acid is at least one selected from the group consisting of formic acid, acetic acid, propionic acid, butyric acid, lactic acid and succinic acid.

12. The intestinal environment improvement composition according to any one of Claims 1 to 11, wherein the glycopeptide is derived from milk.

13. An intestinal environment improvement food and drink containing the intestinal environment improvement composition according to any one of Claims 1 to 12.

14. An intestinal environment improvement pharmaceutical product containing the intestinal environment improvement composition according to any one of Claims 1 to 12.

15. An intestinal environment improvement feed containing the intestinal environment improvement composition according to any one of Claims 1 to 12.

16. A method for producing a composition containing a glycopeptide comprising a peptide having a threonine residue and/or a serine residue and a sugar chain bound to the peptide, wherein
the glycopeptide has a molecular weight of 500 to 3,000,
the sugar chain is bound to a hydroxyl group of the threonine residue or the serine residue of the peptide, and
a constituent sugar of the sugar chain is at least one selected from the group consisting of sialic acid, N-acetylgalactosamine and galactose, the method comprising the following steps of:
(1) treating at least one selected from κ-casein glycomacropeptide, a composition containing κ-casein glycomacropeptide and a composition containing κ-casein, with an endoprotease or an exoprotease, and
(2) subjecting a treated product obtained in the above step to ultrafiltration with a molecular weight cut off of 500 to 3,000.

17. A glycopeptide composition containing a glycopeptide comprising a peptide having a threonine residue and/or a serine residue and a sugar chain bound to the peptide, wherein
the glycopeptide has a molecular weight of 500 to 3,000,
the sugar chain is bound to a hydroxyl group of the threonine residue or the serine residue of the peptide, and
a constituent sugar of the sugar chain is at least one selected from the group consisting of sialic acid, N-acetylgalactosamine and galactose.

18. The glycopeptide composition according to Claim 17, wherein the sugar chain of the glycopeptide contains sialic acid and galacto-N-biose.

19. The glycopeptide composition according to Claim 17 or 18, wherein the sugar chain of the glycopeptide consists of sialic acid and galacto-N-biose.

20. The glycopeptide composition according to any one of Claims 17 to 19, wherein the molar ratio of the sialic acid and the galacto-N-biose is 1.5:1 to 2:1.

21. The glycopeptide composition according to any one of Claims 17 to 20, wherein the total amount of the sialic acid and the galacto-N-biose is 20 wt% or more.

22. The glycopeptide composition according to any one of Claims 17 to 21, wherein the number of amino acid residues in a peptide chain of the glycopeptide is 1 to 13.

23. A glycopeptide composition containing a glycopeptide having a threonine residue and/or a serine residue and a sugar chain bound to a hydroxyl group(s) of the residue(s), wherein the sugar chain has at least one structure selected from the group consisting of the following (a) to (e):
(a) Neu5Acα2-3Galβ1-3(Neu5Acα2-6)GalNAcα1
(b) Galβ1-3(Neu5Acα2-6)GalNAcα1
(c) Neu5Acα2-3Galβ1-3GalNAcα1
(d) Neu5Acα2-3Galβ1-3(O-Ac-Neu5Acα2-6)GalNAcα1
(e) Neu5Acα2-3Galβ1-3(O-diAc-Neu5Acα2-6)GalNAcα1

24. The glycopeptide composition according to Claim 23, wherein the sugar chain has a structure of:
the above (a); and
at least one structure selected from the group consisting of the above (b) to (e).
